# FASCICULE DE BREVET EUROPEEN

(11) **EP 2 262 839 B1**
(45) Date de publication et mention de la délivrance du brevet: **18.01.2012**
(21) Numéro de dépôt: 09742229.9
(22) Date de dépôt: 02.04.2009
(51) Int. Cl.: C07K 16/28, A61K 39/395

(54) **UTILISATION D'UN ANTICORPS ANTI-CD151 POUR LE TRAITEMENT PRECOCE DES CANCERS**
VERWENDUNG EINES ANTIKÖRPERS GEGEN CD151 ZUR FRÜHEN BEHANDLUNG VON KREBS
USE OF AN ANTI-CD151 ANTIBODY FOR EARLY TREATMENT OF CANCER

(30) Priorité: 04.04.2008 FR 0801865
(43) Date de publication de la demande: 22.12.2010
(73) Titulaire: Pierre Fabre Medicament, 92100 Boulogne (FR)
(72) Inventeur: HAEUW, Jean-François, F-74160 Beaumont (FR)
(74) Mandataire: Rousseau, Pierrick Edouard
(86) Numéro de dépôt international: PCT/FR2009/050561
(87) Numéro de publication internationale: WO 2009/136031

(56) Documents cités:
- WO-A-99/66027
- TESTA J E ET AL: "EUKARYOTIC EXPRESSION CLONING WITH AN ANTIMETASTATIC MONOCLONAL ANTIBODY IDENTIFIES A TETRASPANIN (PETA-3/CD151) AS AN EFFECTOR OF HUMAN TUMOR CELL MIGRATION AND METASTASIS" CANCER RESEARCH, AMERICAN ASSOCIATION FOR CANCER RESEARCH, BALTIMORE, MD, vol. 59, no. 15, 1 août 1999 (1999-08-01), pages 3812-3820, XP008064694 ISSN: 0008-5472
- GEARY S M ET AL: "Differential tissue expression of epitopes of the tetraspanin CD151 recognised by monoclonal antibodies" TISSUE ANTIGENS, MUNKSGAARD, COPENHAGEN, DK, vol. 58, no. 3, 1 septembre 2001 (2001-09-01), pages 141-153, XP002435970 ISSN: 0001-2815
- SERRU V ET AL: "Selective tetraspan-integrin complexes (CD81/alpha4beta1, CD151/alpha3beta1, CD151/alpha6beta1) under conditions disrupting tetraspan interactions" BIOCHEMICAL JOURNAL, THE BIOCHEMICAL SOCIETY, LONDON, vol. 340, no. PART 1, 15 mai 1999 (1999-05-15), pages 103-111, XP002435969 ISSN: 0264-6021

## Description

La présente invention est relative à une nouvelle utilisation d'anticorps anti-CD151 capables d'inhiber la croissance tumorale, lesdits anticorps étant notamment monoclonaux d'origine murine, chimérique et humanisée. Selon un aspect particulier, l'invention a pour objet l'utilisation de ces anticorps, ou de leurs fragments fonctionnels, à titre de médicament pour le traitement précoce des cancers et tout particulièrement des tumeurs primaires. L'invention comprend enfin des produits et/ou des compositions comprenant de tels anticorps en association, par exemple, avec des anticorps et/ou des agents anticancéreux ou conjugués avec des toxines et leur utilisation pour la prévention et/ou le traitement de certains cancers.

CD151, appelé aussi PETA-3 ou SFA-1, est une protéine membranaire appartenant à la famille des tétraspanines (Boucheix et Rubinstein, 2001, Cell Mol. Life Sci. 58, 1189-1205 ; Hemler, 2001, J. Cell Biol. 155, 1103-1107). Chez l'homme, CD151 possède 253 acides aminés, et comporte 4 fragments membranaires et 2 domaines extracellulaires EC1 (18 acides aminés, séquence [40-57]) et EC2 (109 acides aminés, séquence [113-221]), appelés aussi boucles extracellulaires. Il est cependant à noter qu'au niveau de la séquence nucléotidique, deux variants pour CD151 ont été identifiés à ce jour, à savoir l'un comprenant les nucléotides A et C respectivement aux positions 395 et 409 [Fitter et al., 1995, Blood 86(4), 1348-1355] et l'autre comprenant pour les mêmes positions les nucléotides G et T au lieu et place des nucléotides A et C [Hasegawa et al., 1996, J. Virol. 70(5), 3258-3263]. De ce fait, une mutation au niveau de la séquence peptidique peut être observée, à savoir une mutation des résidus K (Lys) et P (Pro) respectivement aux positions 132 et 137 en les résidus R Arg) et S (Ser) [Fitter et al., 1995, Blood 86(4), 1348-1355 / Hasegawa et al., 1996, J. Virol. 70(5), 3258-3263].

CD151 est surexprimé dans de nombreux cancers, comme par exemple les cancers du poumon [Tokuhara et al., 2001, Clin. Cancer Res. 7, 4109-4114], du colon [Hashida et al., 2003, Br. J. Cancer 89, 158-167], de la prostate [Ang et al., 2004, Cancer Epidemiol. Biomarkers Prev. 13, 1717-1721] ou du pancréas [Gesierich et al., 2005, Clin. Cancer Res. 11, 2840-2852].

L'utilisation des souris knock-out n'exprimant pas CD151, et d'anticorps anti-CD151 et de siRNA pour bloquer *in vitro* la fonctionnalité et l'expression de CD151 dans différentes types cellulaires a permis de montrer que CD151 est impliqué dans plusieurs phénomènes liés au cancer, comme l'adhésion cellulaire (Nishiuchi et al., 2005, Proc. Natl. Acad. Sci. USA 102, 1939-1944 ; Winterwood et al., 2006, Mol. Biol. Cell 17, 2707-2721), la motilité cellulaire (Kohno et al., 2002, Int. J. Cancer 97, 336-343), la migration cellulaire (Yauch et al., 1998, Mol. Biol. Cell 9, 2751-2765 ; Testa et al., 1999, Cancer Res. 59, 3812-3820 ; Penas et al., 2000, J. Invest. Dermatol, 114, 1126-1135 ; Klosek et al., 2005, Biochem. Biophys. Res. Commun. 336, 408-416), l'invasion cellulaire (Kohno et al., 2002, Int. J. Cancer 97, 336-343 ; Shiomi et al., 2005, Lab. Invest. 85, 1489-1506 ; Hong et al., 2006, J. Biol. Chem. 281, 24279-24292) et l'angiogénèse (Yanez-Mo et al., 1998, J. Cell Biol. 141, 791-804 ; Sincock et al., 1999, J. Cell Sci. 112, 833-844 ; Takeda *et al.,* 2006, Blood).

L'une des propriétés remarquable des tétraspanines est leur capacité à s'associer entre elles, ainsi qu'à un nombre important d'autres molécules de surface pour former des complexes macromoléculaires structurés. Au sein de ces complexes, chaque tétraspanine est associée spécifiquement à une ou plusieurs molécules de surface formant ainsi des complexes primaires, constitués d'une tétraspanine et d'une molécule partenaire. Les tétraspanines peuvent organiser des microdomaines particuliers de la membrane plasmique au sein desquels elles recruteraient leurs partenaires moléculaires qui poudraient être couplés fonctionnellement. L'ensemble des interactions impliquant les tétraspanines a été dénommé « réseau des tétraspanines » ou « Tetraspanin Web ».

CD151 interagit à la surface des cellules avec différentes protéines membranaires. Des complexes très stables, résistants à l'action de certains détergents, ont notamment été mis en évidence avec les intégrines récepteurs des laminines, et plus particulièrement avec les intégrines α3β1 ou α6β4 dont le ligand préférentiel est la laminine 5 (Yauch et al., 1998, Mol. Biol. Cell 9, 2751-2765 ; Lammerding et al., 2003, Proc. Natl. Acad. Sci USA 100, 7616-7621). Cette association implique les domaines extracellulaires de CD151 et des intégrines. La séquence QRD [194-196] de CD151, localisée dans la boucle EC2, est très importante dans cette association puisque la mutation de ce site provoque la perte de l'interaction avec certaines intégrines (Kazarov et al., 2002, J. Cell Biol. 158, 1299-1309). Des complexes ternaires fonctionnels CD151/intéguine α6β4/c-Met (récepteur du HGF) ont d'autre part été mis en évidence dans les cellules tumorales (Klosek et al., 2005, Biochem. Biophys. Res. Commun. 336, 408-416). L'inhibition de l'expression de CD151 par traitement des cellules avec un ARN interférence provoque une inhibition de la croissance et de la migration cellulaire induite par HGF.

Les interactions au sein d'un même cellule de CD151 avec d'autres tétraspanines, nécessaires à la formation du réseau des tétraspanines, dépendraient des régions membranaires et cytoplasmiques de CD151 puisqu'il a été montré que la délétion de la boucle EC2 ne rompait pas l'association de CD151 avec d'autres tétraspanines (Berditchevski, 2001, J. Cell Sci. 114, 4143-4151).

CD151 est capable de réguler les phénomènes d'adhésion, de migration et d'invasion cellulaire par la modulation de différentes voies de signalisation, comme par exemple la voie des phosphoinositides via une association avec la PI4-kinase (Yauch et al., 1998, Mol. Biol. Cell 9, 2751-2765), la voie de signalisation de c-Jun via la phosphorylation de FAK, Src, p38-MAPK et JNK (Hong *et al.,* 2006), la phosphorylation des intégrines par la PKC (Zhang et al., 2001, J. Biol. Chem. 276, 25005-25013), l'activation de GTPases de la famille Rho (Shigeta et al., 2003, J. Cell Biol. 163, 165-176).

Des interactions de type homophilique entre cellules sont aussi responsables d'une augmentation de la motilité cellulaire et de l'expression de la métalloprotéinase MMP-9 (Hong *et al.,* 2006). Ces interactions intercellulaires CD151-CD151 provoquent l'activation de c-Jun via la phosphorylation de FAK, Src, p38-MAPK et JNK

A ce jour, en dépit de l'intérêt de la protéine CD151, deux anticorps à visée thérapeutique ont été générés, à savoir les anticorps monoclonaux 50-6 et SFA1.2B4. Ces 2 anticorps possèdent des activités comparables. Ils inhibent en effet la formation des métastases *in vivo* dans des modèles animaux, mais aucun effet sur la croissance tumorale *in vivo* n'a été mis en évidence.

L'anticorps monoclonal 50-6 (isotype IgG1) dirigé contre CD151 a été généré chez la souris par immunisations soustractives avec des cellules humaines de carcinome épidermoïde HEp-3 (Testa et al., 1999, Cancer Res. 59, 3812-3820).

L'anticorps 50-6 est capable d'inhiber *in vitro* la migration des cellules humaines de carcinome cervical HeLa transfectées afin de surexprimer CD151 et des cellules HEp-3, et l'angiogénèse dans un modèle de néovascularisation de membrane chorioallantoïque induite par le bFGF (basic fibroblast growth factor). Il inhibe *in vivo* les métastases induites par inoculation de cellules HEp-3 dans 2 modèles d'embryon de poulet (Testa et al., 1999, Cancer Res. 59, 3812-3820). Dans ces modèles, l'activité inhibitrice de l'anticorps 50-6 est déterminée par la mesure de l'activité de la proteine huPA (human urokinase-type plasminogen activator) dans des extraits de poumons. Selon les auteurs, ce dosage constitue le reflet de la présence de cellules humaines dans les poumons. Après dosage, la réduction des métastases (dissémination des cellules HEp-3 dans les poumons des embryons de poulet) induite par l'anticorps 50-6 est estimée, par comparaison à un anticorps contrôle, à 74% dans un modèle dit de « métastase spontanée » dans lequel l'inoculation des cellules est suivie par une injection de l'anticorps, et à 57% dans un modèle dit de « métastase expérimentale » dans lequel les cellules et l'anticorps sont inoculés conjointement. Selon les auteurs, les propriétés anti-tumorales de l'anticorps 50-6 observées *in vivo* ne semblent pas être liées à un effet cytostatique ou cytotoxique puisqu'il n'a montré aucun effet sur la prolifération *in vitro* des cellules HEp-3.

L'hybridome produisant l'anticorps 50-6 est disponible à l'ATCC sous la référence CRL-2696 (hybridome déposé initialement sous la référence 50-6 [PTA-227]).

L'anticorps monoclonal anti-CD151 SFA1.2B4 (isotype IgG1) a été généré chez la souris après immunisation par voie intra-péritonéale avec des cellules NIH 3T3 transfectées par le gène de CD151 humain (Hasegawa et al., 1996, J. Virol. 70, 3258-3263). L'anticorps SFA1.2B4 est capable d'inhiber *in vitro* l'invasion et la motilité cellulaire de différentes lignées tumorales humaines (Kohno et al., 2002, Int. J. Cancer 97, 336-343). Il inhibe *in vivo* les métastases pulmonaires induites par les lignées de cancer du colon RPMI14788 et de fibrosarcome HT1080 transfectées pour surexprimer CD151 (Kohno et al., 2002, Int. J. Cancer 97, 336-343).

D'autres anticorps murins anti-CD151 ont été décrits dans la littérature, comme par exemple les anticorps monoclonaux 14A2H1 (Ashman et al., 1991, Br. J. Haematol. 79, 263-270 ; Roberts et al., 1995, Br. J. Haematol. 89, 853-860), TS151 et TS151R (Serru et al., 1999, Biochem. J. 340, 103-111 ; Geary et al., 2001, Tissue Antigens 58, 141-153 ; Charrin et al., J. Biol. Chem. 276, 14329-14337 ; Chometon et al., 2006, Exp. Cell Res. 312, 983-985). Aucune activité anti-tumorale *in vivo* n'a été décrite pour ces différents anticorps.

Selon un premier aspect, l'invention vise l'utilisation d'un anticorps monoclonal sécrété par l'hybridome déposé à l'ATCC sous la référence CRL-2696, ou l'un de ses fragments fonctionnels, pour la préparation d'un médicament destiné à inhiber la croissance tumorale d'une tumeur primaire pour le traitement précoce du cancer. Plus particulièrement, ledit anticorps est l'anticorps monoclonal 50-6. Selon encore un aspect de l'invention, l'anticorps dont l'utilisation fait l'objet de l'invention est spécifique de la protéine CD 151.

Contrairement à ce qui était connu sur ledit anticorps 50-6, la demanderesse a mis en évidence pour la première fois que cet anticorps présentait une activité d'inhibition de la croissance tumorale et, tout particulièrement, de la croissance d'une tumeur primaire. Contre toute attente, l'invention vise donc une utilisation de cet anticorps pour la préparation d'une composition pharmaceutique visant à traiter précocement le cancer.

Par « traitement précoce » ou « traiter précocement », il faut comprendre un traitement inhibant une étape précoce du processus de développement de la tumeur avant l'apparition de métastases par opposition aux étapes tardives se produisant une fois le processus métastatique établi. A titre d'exemple non limitatif, il peut s'agir de l'inhibition de la prolifération des cellules tumorales au niveau de la tumeur primaire, donc antérieurement au processus métastatique.

Plusieurs études expérimentales ont montré le rôle majeur des tétraspanines dans la formation de métastases, en agissant soit comme suppresseurs, soit comme promoteurs de métastases. Ainsi, la transfection de tétraspanines comme CD9, CD63 ou CD82 réduit le potentiel métastatique de lignées cancéreuses. En revanche, l'expression des tétraspanines CD151 et Co-029 semble produire l'effet inverse. Ces 2 tétraspanines seraient donc des promoteurs de la métastase. Ces résultats sont cohérents avec différentes études cliniques qui ont démontré que, dans plusieurs cancers (sein, poumon, oesophage, estomac, foie, pancréas, côlon, prostate, mélanome...), CD9 et CD82 sont moins exprimés sur les tumeurs primitives lorsqu'il y a métastase et que leur baisse d'expression prédit un taux de survie inférieur. Dans le cancer du poumon, la diminution combinée de l'expression de CD9 et de CD82 a été corrélée à un potentiel métastatique plus important que lorsque l'expression d'un seul de ces deux antigènes est réduite.

Plusieurs études rétrospectives ont montré que la surexpression de CD151 était associée à l'agressivité de certains cancers, comme les cancers du poumon, du colon et de la prostate, et pouvait être considérée comme un facteur de mauvais pronostic (Tokuhara et al., 2001, Clin. Cancer Res. 7, 4109-4114 ; Hashida et al., 2003, Br. J. Cancer 89, 158-167 ; Ang et al., 2004, Cancer Epidemiol. Biomarkers Prev. 13, 1717-1721). Dans ces cas, la moyenne de survie est en effet diminuée chez les patients dont la tumeur exprime CD151, comparativement à ceux dont la tumeur n'exprime pas CD151.

La surexpression de CD151 dans différentes lignées tumorales humaines (HeLa, RPMI14788, A172, HT1080), induite par transfection du gène correspondant, provoque une augmentation de la motilité, de la migration et de l'invasion des cellules transfectées (Testa et al., 1999, Cancer Res. 59, 3812-3820 ; Kohno et al., 2002, Int. J. Cancer 97, 336-343). Ces phénomènes sont inhibés en présence d'anticorps anti-CD151.

Selon un autre aspect, les fragments fonctionnels d'anticorps selon l'invention consistent, par exemple, en des fragments Fv, scFv (sc pour simple chaîne), Fab, F(ab')₂, Fab', scFv-Fc ou diabodies, ou tout fragment dont la durée de demie-vie aurait été augmentée par modification chimique, comme l'ajout de poly(alkylène) glycol tel que le poly(éthylène)glycol ("PEGylation") (fragments PEGylés appelés Fv-PEG, scFv-PEG, Fab-PEG, F(ab')₂-PEG ou Fab'-PEG) (« PEG » d'après la nomenclature anglaise Poly(Ethylene) Glycol), ou par incorporation dans un liposome, microsphères ou PLGA, lesdits fragments étant capables d'exercer de manière générale une activité même partielle de l'anticorps dont ils sont issus.

De préférence, lesdits fragments fonctionnels seront constitués ou comprendront une séquence partielle de la chaîne variable lourde ou légère de l'anticorps dont ils sont dérivés, ladite séquence partielle étant suffisante pour retenir la même spécificité de liaison que l'anticorps dont elle est issue et une affinité suffisante, de préférence au moins égale à 1/100, de manière plus préférée à au moins 1/10 de celle de l'anticorps dont elle est issue.

Un tel fragment fonctionnel comportera au minimum 5 acides aminés, de préférence 10, 15, 25, 50 et 100 acides aminés consécutifs de la séquence de l'anticorps dont il est issu.

De préférence, ces fragments fonctionnels seront des fragments de type Fv, scFv, Fab, F(ab')₂, F(ab'), scFv-Fc ou diabodies, qui possèdent généralement la même spécificité de fixation que l'anticorps dont ils sont issus. Selon la présente invention, des fragments d'anticorps de l'invention peuvent être obtenus à partir des anticorps tels que décrits précédemment par des méthodes telles que la digestion par des enzymes, comme la pepsine ou la papaïne et/ou par clivage des ponts disulfures par réduction chimique. D'une autre manière les fragments d'anticorps compris dans la présente invention peuvent être obtenus par des techniques de recombinaisons génétiques bien connues également de l'homme de l'art ou encore par synthèse peptidique au moyen par exemple de synthétiseurs automatiques de peptides tels que ceux fournis par la société Applied.

Selon un aspect de l'invention, l'anticorps utilisé consiste en un anticorps monoclonal murin.

Sont également compris par anticorps selon la présente invention, les anticorps chimériques ou humanisés.

Par anticorps chimérique, on entend désigner un anticorps qui contient une région variable (chaîne légère et chaîne lourde) naturelle dérivée d'un anticorps d'une espèce donnée en association avec les régions constantes de chaîne légère et chaîne lourde d'un anticorps d'une espèce hétérologue à ladite espèce donnée.

Les anticorps ou leurs fragments de type chimérique utilisés selon l'invention peuvent être préparés en utilisant les techniques de recombinaison génétique. Par exemple, l'anticorps chimérique pourra être réalisé en clonant un ADN recombinant comportant un promoteur et une séquence codant pour la région variable d'un anticorps monoclonal non humain, notamment murin, selon l'invention et une séquence codant pour la région constante d'anticorps humain. Un anticorps chimérique de l'invention codé par un tel gène recombinant sera par exemple une chimère souris-homme, la spécificité de cet anticorps étant déterminée par la région variable dérivée de l'ADN murin et son isotype déterminé par la région constante dérivée de l'ADN humain. Pour les méthodes de préparation d'anticorps chimériques, on pourra par exemple se référer au document Verhoeyn et al. (BioEssays, 8:74, 1988).

Par anticorps humanisés, on entend désigner un anticorps qui contient des régions CDRs dérivées d'un anticorps d'origine non humaine, les autres parties de la molécule d'anticorps étant dérivée d'un (ou de plusieurs) anticorps humains. En outre, certains des résidus des segments du squelette (dénommés FR) peuvent être modifiés pour conserver l'affinité de liaison (Jones et al., Nature, 321:522-525, 1986 ; Verhoeyen et al., Science, 239:1534-1536, 1988 ; Riechmann et al., Nature, 332:323-327, 1988).

Les anticorps humanisés ou leurs fragments fonctionnels peuvent être préparés par des techniques connues de l'homme de l'art (comme par exemple celles décrites dans les documents Singer et al., J. Immun. 150:2844-2857, 1992 ; Mountain et al., Biotechnol. Genet. Eng. Rev., 10:1-142, 1992 ; ou Bebbington et al., Bio/Technology, 10:169-175, 1992). De tels anticorps humanisés sont préférés pour leur utilisation dans des méthodes de traitement prophylactique et/ou thérapeutique *in vivo.* D'autres techniques d'humanisation sont également connues de l'homme de l'art comme par exemple la technique du *« CDR Crafting* » décrite par PDL faisant l'objet des brevets EP 0 451 261, EP 0 682 040, EP 0 939 127, EP 0 566 647 ou encore US 5,530,101, US 6,180,370, US 5,585,089 et US 5,693,761. On peut également citer les brevets US 5,639,641 ou encore 6,054,297, 5,886,152 et 5,877,293.

Plus particulièrement, la demanderesse avance, sans vouloir être liée par une quelconque théorie, que l'utilisation d'anticorps anti-CD151 dans le cadre du traitement du cancer présenterait un intérêt, non pas uniquement du fait d'une inhibition de l'angiogénèse, mais également et surtout du fait de l'inhibition de l'activité promotrice de métastase de CD151. En effet, contrairement à ce qui était connu à ce jour, à savoir un effet de l'anticorps 50-6 sur l'angiogénèse, la migration et l'invasion, l'utilisation selon l'invention vise le phénomème même de croissance tumorale des tumeurs primaires (avec comme conséquence directe évidente une inhibition de la formation de métastases et non pas uniquement du processus métastatique, et notamment de l'adhésion cellulaire, de la migration cellulaire et/ou de l'invasion cellulaire.

Comme cela a été dit plus haut, la protéine CD151 fait partie de la famille des tétraspanines et, à ce titre, comprend 2 domaines extracellulaires EC1 (18 acides aminés, séquence [40-57]) et EC2 (109 acides aminés, séquence [113-221]), appelés aussi boucles extracellulaires.

Selon la présente invention, les anticorps utilisés sont capables de se lier à au moins un épitope situé dans le domaine extracellulaire. De manière préférée, ledit anticorps se fixera au niveau des boucles EC1 et/ou EC2.

Plus particulièrement, selon une forme d'exécution préférée de l'invention, il est décrit l'utilisation d'au moins un anticorps anti-CD151, ou l'un de ses fragments fonctionnels, capable de se lier à un épitope compris dans la boucle extra-cellulaire 1 (EC1) et/ou 2 (EC2), préférentiellement EC2, correspondant respectivement aux acides-aminés 40-57 et 113-221 de la protéine CD151.

La boucle EC1 [40-57] comprend 18 acides-aminés et présente une masse théorique de 2002,2 Da.

La boucle EC2 [113-221] possède un site de N-glycosylation (résidu Asn159) et 6 résidus Cystéine formant 3 ponts disulfure. Un modèle structural de la boucle EC2 des tétraspanines, et notamment de CD151, a été proposé à partir de la structure tridimensionnelle de la boucle EC2 de la tétraspanine CD81 (Seigneuret et al., 2001, J. Biol. Chem. 276, 40055-40064). Selon ce modèle, les tétraspanines possèdent une charpente commune, relativement conservée et constituée de 3 hélices α, et un domaine spécifique variable. Pour CD151, cette charpente serait constituée des régions [113-157] et [209-221], et le domaine variable de la région [158-208].

Le domaine variable de la boucle EC2 serait plus particulièrement impliqué dans les interactions spécifiques de CD151 avec des protéines de la famille des intégrines. Des expériences de mutagénèse dirigée ont notamment montré l'importance de la région [193-208], et plus précisément du tripeptide QRD [194-196] et du résidu Cystéine en position 192, dans l'association de CD151 avec certaines intégrines récepteurs de la laminine, telles que les intégrines α3β1 ou α6β4 (Kazarov et al., 2002, J. Cell Biol. 158, 1299-1309).

Il faut entendre par « anticorps monoclonal » un anticorps issu d'une population d'anticorps sensiblement homogènes. Plus particulièrement, les anticorps individuels d'une population sont identiques à l'exception de quelques mutations éventuelles pouvant se produire naturellement qui peuvent se retrouver en proportions minimes. En d'autres termes, un anticorps monoclonal consiste en un anticorps homogène résultant de la prolifération d'un seule clone cellulaire (par exemple un hybridome, une cellule hôte eucaryote transféctée avec une molécule d'ADN codant pour l'anticorps homogène, une cellule hôte procaryote transféctée avec une molécule d'ADN codant pour l'anticorps homomgène, etc.) et qui est généralement caractérisé par des chaînes lourdes d'une seule et même classe et sous-classe, et des chaînes légères d'un seul type. Les anticorps monoclonaux sont fortement spécifiques et sont dirigés contre un seul antigène. En outre, contrairement aux préparations d'anticorps polyclonaux qui comprennent classiquement différents anticorps dirigés contre différents déterminants, ou épitopes, chaque anticorps monoclonal est dirigé contre un seul épitope de l'antigène.

De manière préférée, l'utilisation des anticorps anti-CD151 dans le cadre du traitement du cancer se justifie tout particulièrement dans les cancers surexprimant ce même récepteur CD 151.

De tels cancers consistent en le cancer du colon [Hashida et al., Br. J. Cancer 89 (2003) :158-167], le cancer du poumon, préférentiellement du poumon non à petites cellules [Tokuhara et al., Clin. Cancer Res. 7 (2001) :4109-4114], le cancer de la prostate [Ang et al., Cancer Epidemiol. Biomarkers 13 (2004) :17] et le cancer du pancréas [Gesierich et al., Clin. Cancer Res. 11 [2005] :2840-2852].

La présente invention revendique donc l'utilisation d'un anticorps tel que décrit plus haut pour le traitement du cancer, ledit cancer consistant préférentiellement en les cancers du colon, du poumon, de la prostate ou du pancréas.

L'invention concerne également une composition pharmaceutique comprenant à titre de principe actif un composé consistant en un anticorps, ou l'un de ses composés dérivés ou fragments fonctionnels, de préférence additionné d'un excipient et/ou d'un véhicule pharmaceutiquement acceptable.

Plus particulièrement, l'invention vise l'utilisation d'un anticorps selon l'invention pour la préparation d'une composition pharmaceutique comprenant, en outre, au moins un véhicule pharmaceutiquement acceptable

Dans la présente description, on entend désigner par véhicule pharmaceutiquement acceptable, un composé ou une combinaison de composés entrant dans une composition pharmaceutique ne provoquant pas de réactions secondaires et qui permet par exemple la facilitation de l'administration du ou des composés actifs, l'augmentation de sa durée de vie et/ou de son efficacité dans l'organisme, l'augmentation de sa solubilité en solution ou encore l'amélioration de sa conservation. Ces véhicules pharmaceutiquement acceptables sont bien connus et seront adaptés par l'homme de l'art en fonction de la nature et du mode d'administration du ou des composés actifs choisis.

De préférence, ces composés seront administrés par voie systémique, en particulier par voie intraveineuse, par voie intramusculaire, intradermique, intrapéritonéale ou sous-cutanée, ou par voie orale. De manière plus préférée, la composition comprenant les anticorps selon l'invention, sera administrée à plusieurs reprises, de manière étalée dans le temps.

Leurs modes d'administration, posologies et formes galéniques optimaux peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés.

Selon l'invention, il est décrit une composition pour le traitement du cancer, caractérisée en ce qu'elle comprend à titre de principe actif au moins un anticorps monoclonal sécrété par l'hybridome déposé à l'ATCC sous la référence CRL-2696, ou l'un de ses fragments fonctionnels.

Plus particulièrement, il est décrit une composition comprenant au moins un anticorps anti-CD151, ou l'un de ses fragments fonctionnels, ledit anticorps monoclonal consistant en l'anticorps 50-6.

Il ressort de la littérature que la protéine CD151 est surexprimée dans les cancers et, tout particulièrement, dans les carcinomes du colon [Hashida et al., Br. J. Cancer 89 (2003): 158-167], les cancers du poumon non à petites cellules [Tokuhara et al., Clin. Cancer Res. 7 (2001): 4109-4114], les cancers de la prostate [Ang et al., Cancer Epidemiol. Biomarkers 13 (2004): 1717-1721] et les cancers du pancréas [Gesierich et al., Clin. Cancer Res. 11 (2005): 2840-2852].

Bien évidemment, la liste ci-dessus n'est donnée qu'à titre illustratif et tout cancer doit être compris comme surexprimant la protéine CD 151 et donc susceptible d'être traité conformément à la présente invention. Une autre forme d'exécution complémentaire de l'invention consiste en une composition telle que décrite plus haut qui comprend, en outre, comme produit de combinaison pour une utilisation simultanée, séparée ou étalée dans le temps, un agent cytotoxique/cytostatique.

La présente invention concerne donc également une composition telle que décrite plus haut, caractérisée en ce qu'elle comprend, en outre, comme produit de combinaison pour une utilisation simultanée, séparée ou étalée dans le temps, au moins un agent cytotoxique/cytostatique et/ou une toxine cellulaire et/ou un radioélément.

On entend par "utilisation simultanée", l'administration des deux composés de la composition selon l'invention compris dans une seule et même forme pharmaceutique.

On entend par "utilisation séparée", l'administration, en même temps, des deux composés de la composition selon l'invention, compris dans des formes pharmaceutiques distinctes.

On entend par "utilisation étalée dans le temps", l'administration successive des deux composés de la composition selon l'invention, compris chacun dans une forme pharmaceutique distincte.

D'une façon générale, la composition selon l'invention augmente considérablement l'efficacité du traitement du cancer. En d'autres termes, l'effet thérapeutique de l'anticorps selon l'invention est potentialisé de manière inattendue par l'administration d'un agent cytotoxique. Un autre avantage subséquent majeur produit par une composition selon l'invention, concerne la possibilité d'utiliser des doses efficaces en principe actif plus faibles, ce qui permet d'éviter ou de réduire les risques d'apparition des effets secondaires, en particulier l'effet de l'agent cytotoxique. De plus, cette composition selon l'invention permettrait d'atteindre l'effet thérapeutique escompté plus rapidement.

Par « agents thérapeutiques anti-cancer » ou « agents cytotoxiques », il faut comprendre une substance qui, lorsqu'elle est administrée à un patient, traite ou prévient le développement du cancer chez le patient. A titre d'exemple non limitatif pour de tels agents, il peut être mentionné les agents « alkylant », les antimétabolites, les antibiotiques anti-tumoraux, les inhibiteurs mitotiques, les inhibiteurs de fonction chromatine, les agents anti-angiogénèse, les anti-oestrogène, les anti-androgène ou les immuno-modulateurs.

De tels agents sont, par exemple, cités dans le VIDAL, à la page consacrée aux composés attachés à la cancérologie et l'hématologie colonne « Cytotoxiques », ces composés cytotoxiques cités par référence à ce document sont cités ici comme agents cytotoxiques préférés.

Les « agents alkylants » font référence à toute substance qui peut se coupler de manière covalente ou alkyler toute molécule, préférentiellement un acide nucléique (ex. : ADN), au sein d'une cellule. Comme exemples de tels agents alkylants, il peut être cité les moutardes à l'azote comme la méchloréthamine, le chlorambucil, le melphalan, le chlorhydrate, le pipobroman, la prednimustine, le phosphate disodique ou l'estramustine ; les oxazaphosphorines comme la cyclophosphamide, l' altretamine, la trofosfamide, la sulfofosfamide ou l'ifosfamide ; les aziridines ou ethylènes-imines comme le thiotepa, la triéthylèneamine ou l'altétramine ; les nitrosourées comme la carmustine, la streptozocine, la fotémustine ou la lomustine ; les sulfonates d'alkyle comme le busulfan, le tréosulfan ou l'improsulfan ; les triazènes comme la dacarbazine ; ou encore les complexes du platine comme le cisplatine, l'oxaliplatine ou le carboplatine.

Les « antimétabolites » font référence à des substances qui bloquent la croissance et/ou le métabolisme cellulaire en interférant avec certaines activités, généralement la synthèse d'ADN. A titre d'exemple d'antimétabolite, il peut être mentionné les methotrexate, 5-fluorouracile, floxuridine, 5-fluorodeoxyuridine, capecitabine, cytarabine, fludarabine, cytosine arabinoside, 6-mercaptopurine (6-MP), 6-thioguanine (6-TG), chlorodésoxyadénosine, 5-azacytidine, gemcitabine, cladribine, deoxycoformycine et la pentostatine.

Les « antibiotiques anti-tumoraux » font référence aux composés qui peuvent prévenir ou inhiber la synthèse d'ADN, d'ARN et/ou de protéines. Des exemples de tels antibiotiques anti-tumoraux comprennent la doxorubicine, daunorubicine, idarubicine valrubicine, mitoxantrone, dactinomycine, mithramycine, plicamycine, mitomycine C, bleomycine, et la procarbazine.

Les « inhibiteurs mitotiques » préviennent la progression normale du cycle cellulaire et de la mitose. En general, les inhibiteurs des microtubules ou « taxoides » comme le paclitaxel et le docétaxel sont capables d'inhiber la mitose. Les alcaloîdes de vinca, comme la vinblsatine, la vincristine, la vindésine et la vinorelbine sont également capables d'inhiber la mitose.

Les « inhibiteurs de fonction chromatine » ou « inhibiteurs de topo-isomérases » font référence à des substances qui inhibent la fonction normale des protéines modélant la chromatine comme les topo-isomérases I et II. Des exemples de tels inhibiteurs comprennent, pour la topo-isomérase I, la camptothécine ainsi que ses dérivés comme l'irinotécan ou le topotécan et, pour la topo-isomérase II, l'étoposide, le phosphate d'étiposide et le téniposide.

Les « agents anti-angiogénèse » font références à toute drogue, composé, substance ou agent qui inhibe la croissance des vaisseaux sanguins. Des exemples d'agents anti-angiogénèse comprennent, sans aucune limitation, les razoxin, marimastat, batimastat, prinomastat, tanomastat, ilomastat, CGS-27023A, halofuginone, COL-3, neovastat, BMS-275291, thalidomide, CDC 501, DMXAA, L-651582, squalamine, endostatine, SU5416, SU6668, interferon-alpha, EMD121974, interleukine-12, IM862, angiostatine et la vitaxine.

Les « anti-oestrogène » ou « agents anti-oestrogénique » font référence à toute substance qui diminue, antagonise ou inhibe l'action des oestrogènes. Des exemples de tels agents sont les tamoxifène, toremifène, raloxifène, droloxifène, iodoxyfène, anastrozole, letrozole, et l'exemestane.

Les « anti-androgènes » ou « agents anti-androgène » font référence à toute substance qui réduit, antagonise ou inhibe l'action d'un androgène. Des exemples d'anti-androgènes sont les flutamide, nilutamide, bicalutamide, sprironolactone, cyproterone acetate, finasteride et la cimitidine.

Les immunomodulateurs sont des substances qui stimulent le système immunitaire. Des exemples de tels immunomodulateurs comprennent les interférons, les interleukines comme l'aldesleukine, OCT-43, denileukin diflitox ou l'interleukine-2, les facteurs de nécrose tumorale comme la tasonermine, ou d'autres types d'immunomodulateurs comme le lentinan, le sizofiran, le roquinimex, le pidotimod, la pégadémase, la thymopentine, le poly I :C, ou le levamisole en combinaison avec le 5-fluorouracil.

Pour plus de détails, l'homme de l'art pourra se reporter au manuel édité par l'Association Française des Enseignants de Chimie Thérapeutique intitulé « traité de chimie thérapeutique, Vol. 6, Médicaments antitumoraux et perspectives dans le traitement des cancers, édition TEC & DOC, 2003 ».

Dans un mode de réalisation particulièrement préféré, ladite composition comme produit de combinaison selon l'invention est caractérisée en ce que ledit agent cytotoxique est couplé chimiquement audit anticorps pour une utilisation simultanée.

Dans un mode de réalisation particulièrement préféré, ladite composition selon l'invention est caractérisée en ce que ledit agent cytotoxique/cytostatique est choisi parmi les agents inhibiteurs ou stabilisateurs du fuseau, de préférence la vinorelbine et/ou la vinflunine et/ou la vincristine.

Afin de faciliter le couplage entre ledit agent cytotoxique et ledit anticorps selon l'invention, on pourra notamment introduire des molécules espaceurs entre les deux composés à coupler, telles que des poly(alkylènes)glycols comme le polyéthylèneglycol, ou encore des acides aminés, ou, dans un autre mode de réalisation, utiliser des dérivés actifs desdits agents cytotoxiques dans lesquels auront été introduites des fonctions capables de réagir avec ledit anticorps selon l'invention. Ces techniques de couplage sont bien connues de l'homme de l'art et ne seront pas développées dans la présente description.

L'invention est, sous un autre aspect, relative à une composition caractérisée en ce que l'un, au moins, desdits anticorps, ou l'un de leur composé dérivé ou fragment fonctionnel, est conjugué avec une toxine cellulaire et/ou un radioélément

De préférence, ladite toxine ou ledit radioélément est capable d'empêcher la croissance ou la prolifération de la cellule tumorale, notamment d'inactiver totalement ladite cellule tumorale.

De préférence encore, ladite toxine est une toxine d'entérobactéries, notamment l'exotoxine A de Pseudomonas.

Les radioéléments (ou radio-isotopes) préférentiellement conjugués à l'anticorps employés en thérapie sont des radio-isotopes qui émettent des rayons gamma et préférentiellement l'iodine¹³¹, l'yttrium⁹⁰, l'or¹⁹⁹, le palladium¹⁰⁰, le cuivre⁶⁷, le bismuth²¹⁷ et l'antimony²¹¹. Les radio-isotopes qui émettent des rayons beta et alpha peuvent également être utilisés en thérapie.

Par toxine ou radioélément conjugué à au moins un anticorps, ou l'un de leur fragment fonctionnel, selon l'invention, on entend désigner tout moyen permettant de lier ladite toxine ou ledit radioélément audit au moins un anticorps, notamment par couplage covalent entre les deux composés, avec ou sans introduction de molécule de liaison.

Parmi les agents permettant une liaison chimique (covalente), électrostatique ou non covalente de tout ou partie des éléments du conjugué, il peut être mentionné tout particulièrement le benzoquinone, le carbodiimide et plus particulièrement l'EDC (1-ethyl-3-[3-dimethylaminopropyl]-carbodiimide hydrochloride), le dimaleimide, l'acide dithiobis-nitrobenzoic (DTNB), le N-succinimidyl S-acetyl thio-acetate (SATA), les agents dits de « bridging » présentant un ou plusieurs groupements, ayant un ou plusieuyrs groupements phenylaside, réagissant avec les ultraviolets (U.V.) et tout préférentiellement le N-[-4-(azidosalicylamino)butyl]-3'-(2'-pyridyldithio)propionamide (APDP), le N-succinimid-yl 3-(2-pyridyldithio)propionate (SPDP) et le 6-hydrazinonicotinamide (HYNIC).

Une autre forme de couplage, tout spécialement pour les radioéléments, peut consister en l'utilisation d'un chélateur d'ions bifonctionnel.

Parmi ces chélateurs, il est possible de mentionner les chélates dérivés de l'EDTA (ethylenediaminetetraacetic acid) ou du DTPA (diethylenetriaminepentaacetic acid) qui ont été développés pour lier des métaux, particulièrement des métaux radioactifs, et des immunoglobulines. Ainsi, le DTPA et ses dérivés peut être substitué par différents groupements sur le chaîne de carbones de manière à augmenter la stabilité et la rigidité du complexe ligand-métal (Krejcarek et al. (1977); Brechbiel et al. (1991); Gansow (1991); US patent 4 831 175).

Par exemple, le DTPA (diethylenetriaminepentaacetic acid) et ses dérivés, qui a été très largement utilisé en médecine et en biologie pendant longtemps soit sous sa forme libre, soit sous la forme d'un complexe avec un ion métallique, présente la caractéristique remarquable de former des chélates stables avec des ions métalliques et d'être couplé à des protéines d'intérêt thérapeutique ou diagnostique comme des anticorps pour le développement de radio-immunoconjugués en thérapie du cancer (Meases et al., (1984); Gansow et al. (1990)).

L'invention concerne également une composition comprenant l'anticorps selon l'invention, ladite composition étant caractérisée en ce qu'elle comprend, en outre, au moins un deuxième anticorps anti-tumoral.

La présente invention comprend en outre l'utilisation de la composition selon l'invention pour la préparation d'un médicament.

La présente invention vise donc plus particulièrement l'utilisation d'une composition telle que décrite plus haut pour la préparation d'un médicament destiné au traitement du cancer. Parmi les cancers qui peuvent être prévenus et/ou traités, on préfère le cancer du colon, du poumon, de la prostate ou du pancréas.

L'invention a également pour objet l'utilisation d'un anticorps selon l'invention, pour la préparation d'un médicament destiné au ciblage spécifique d'un composé biologiquement actif vers des cellules exprimant ou surexprimant le récepteur CD151.

On entend désigner ici par composé biologiquement actif tout composé capable de moduler, notamment d'inhiber, l'activité cellulaire, en particulier leur croissance, leur prolifération, la transcription ou la traduction de gène.

D'autres caractéristiques et avantages de l'invention apparaissent dans la suite de la description avec les exemples et les figures dont les légendes sont représentées ci-après.

### LEGENDES DES FIGURES

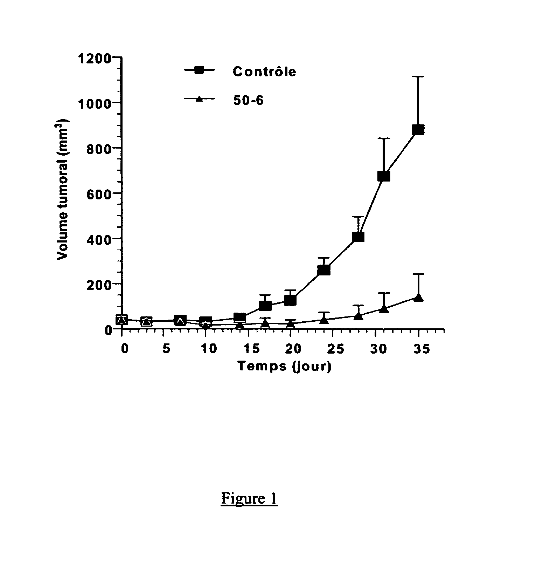

La figure 1 illustre l'activité anti-tumorale *in vivo* de l'anticorps 50-6 dans un modèle de xénogreffe de cancer de la prostate. Des souris Swiss Nude (n=6) ont été greffées par voie sous cutanée avec les cellules PC3. Cing jours après la greffe des cellules, les souris reçoivent, par voie i.p., une dose d'appel de 2 mg/souris de l'anticorps à tester suivie de deux administrations par semaine d'une dose de 1 mg/souris de cet anticorps. Le volume tumoral est évalué par la formule π/6 x Longueur x largeur x épaisseur et un test de Mann et Whitney est effectué pour l'évaluation statistique des résultats.

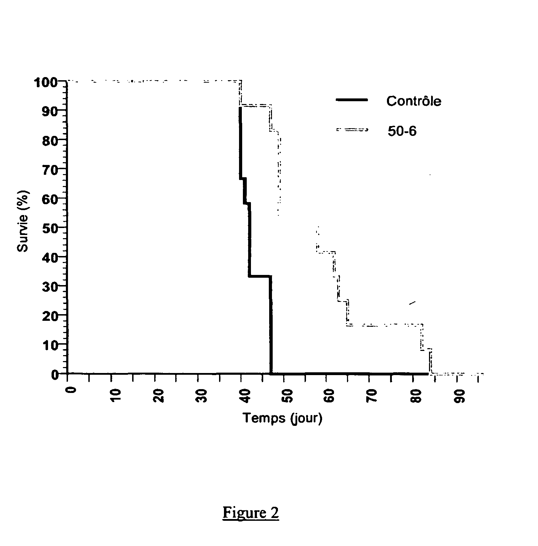

La figure 2 illustre l'évaluation de l'activité anti-tumorale de l'anticorps TS151 in vivo dans un modèle orthotopique de cancer du poumon. Des souris immunodéprimées (n=10) sont greffées par voie intrapleurale avec 1.10⁶ cellules A549. Sept jours après la greffe, les souris sont traitées par voie intrapéritonéale avec une dose d'appel de 2 mg d'anticorps TS151 suivie d'un traitement, deux fois par semaine, durant 5 semaines, avec une dose de 1 mg d'anticorps par souris. Le lot contrôle est injecté avec du PBS selon le même schéma d'administration.

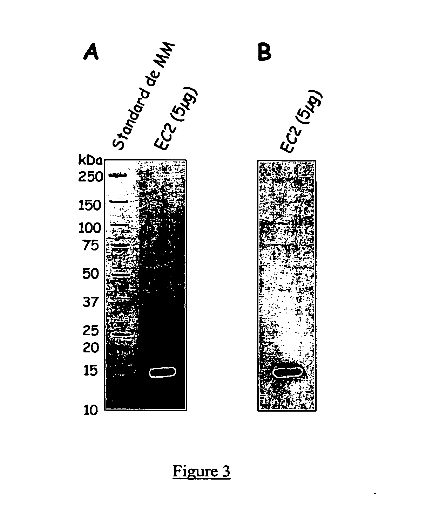

La figure 3 illustre l'évaluation de la spécificité de l'anticorps 50-6 par western blot.Après électrophorèse SDS-PAGE, le gel a été coloré au bleu de Coomassie (A) ou les protéines ont été transférées sur membrane de nitrocellulose afin de réaliser un western blot (B). Dans ce cas, la membrane de transfert a ensuite été incubée avec l'anticorps 50-6 (1 µg/ml), puis avec un anticorps polyclonal de lapin anti-Ig de souris couplé à la péroxydase (GE Healthcare) avant révélation par chimioluminescence.

### Exemple 1 : Effet de l'anticorps 50-6 sur la croissance in vivo de la tumeur PC3 implantée en sous cutanée chez la souris Nude

Une surexpression de CD151 dans les tissus tumoraux de cancer de la prostate ayant été observée par immunohistochimie, une évaluation de l'anticorps anti-CD151 50-6 sur une xénogreffe de cellules de cancer de la prostate PC3 a été envisagée. La lignée PC3 est une lignée prostatique androgéno-indépendante originaire de l'ATCC et cultivée en milieu F12K+10% SVF+L-Glutamine. Pour l'évaluation, 5.10⁶ cellules PC3 sont implantées sur le flanc droit de souris Swiss Nude. Cinq jours après implantation, les animaux sont randomisés sur la base du volume tumoral et répartis en 2 groupes comparables. Le volume tumoral des animaux greffés sélectionnés est compris entre 41 et 47 mm³ (volume calculé avec la formule π/6 x longueur x largeur x épaisseur) au jour 0 du traitement. Les animaux reçoivent alors l'anticorps purifié à tester ou du PBS (groupe contrôle) par voie intra-péritonéale. Les doses d'anticorps et la fréquence des injections sont les suivantes : dose d'appel 2 mg/dose d'anticorps ; dose de maintien 1 mg/dose 2 fois par semaine.

Les résultats présentés à la figure 1 montrent que l'anticorps 50-6 inhibe significativement la croissance de la tumeur PC3 implantée en position sous-cutanée chez la souris Swiss Nude. Une analyse statistique montre que cette inhibition de croissance tumorale est significative, par rapport au groupe contrôle, à partir du jour 10 de traitement (p ≤ 0,01).

### Exemple 2 : Evaluation de l'activité anti-tumorale de l'anticorps 50-6 dans un modèle orthotopique de cancer du poumon

Les cellules A549 originaires de l'ATCC sont cultivées en routine en milieu F12K, 10 mM de glutamine, 10% de SVF. Ces cellules sont divisées 2 jours avant la greffe afin qu'elles soient en phase exponentielle de croissance. Pour la greffe, des souris immunodéprimées de 7 semaines sont anesthésiés avant de recevoir 1.10⁶ cellules A549 par voie intra-pleurale. La tumeur primaire se développe rapidement et envahie en 4 jours les structures adjacentes au site d'injection incluant le médiastin, les poumons et le diaphragme. Pour mieux mimer la maladie, le début du traitement ne commence que 7 jours après implantation des cellules, par voie intra-péritonéale. Après injection d'une dose d'appel de 2 mg/souris, l'anticorps 50-6 est administré 2 fois par semaine, durant 5 semaines à la dose de 1 mg/souris. Un groupe de souris recevant du PBS est introduit comme contrôle étant donné que des expériences effectuées précédemment ont montré que l'administration d'un isotype contrôle IgG1 était sans aucun impact sur la survie des animaux.

Le paramètre d'évaluation de ce modèle est la survie des animaux et l'activité anti-tumorale est exprimée par le calcul du T/C% = médiane de survie des animaux traités/médiane de survie des animaux du groupe control X 100. Il a été établi qu'un T/C% supérieur ou égale à 125 % signe une activité du produit.

La figure 2 montre une activité anti-tumorale de l'anticorps 50-6 avec un T/C% calculé de 127% (p < 0,001). Ce résultat confirme l'activité anti-métastatique de l'anticorps 50-6.

### Exemple 3 : Spécificité de l'anticorps 50-6

La spécificité de l'anticorps 50-6 a été évaluée par western blot. La protéine recombinante EC2 CD151 (5 µg) a été déposée sur un gel d'acrylamide 4-12% (BioRad). Après électrophorèse (conditions non réductrices), les protéines ont été transférées sur membrane de nitrocellulose. Les membranes de transfert ont ensuite été incubées avec les anticorps 50-6 purifiés (1 µg/ml), puis avec un anticorps polyclonal de lapin anti-Ig de souris couplé à la péroxydase (GE Healthcare) avant révélation par chimioluminescence.

La boucle EC2 de CD151 a été clonée dans le vecteur pET22b pour une expression sous forme soluble dans le périplasme de Escherichia coli. La protéine produite comporte les acides aminés 130 à 221 de la séquence peptidique de CD151 humain, auxquels a été ajoutée une queue Poly-His en position C-terminale pour faciliter la purification. La protéine EC2 recombinante a été purifiée par chromatographie d'affinité sur métaux immobilisés (IMAC) sur support Chelating Sepharose HP (GE Healthcare).

La figure 3 montre que l'anticorps anti-CD151 50-6 reconnaît spécifiquement la boucle EC2 par western blot. Cet anticorps est conformationnel car on constate une perte de reconnaissance lorsque le western blot est réalisé après une analyse SDS-PAGE en conditions réductrices.

## Revendications

1. Utilisation d'un anticorps monoclonal capable de se lier spécifiquement à la protéine CD151, sécrété par l'hybridome déposé à l'ATCC sous la référence CRL-2696, ou l'un de ses fragments fonctionnels, pour la préparation d'un médicament destiné au traitement des tumeurs primaires pour le traitement précoce du cancer.

2. Utilisation selon la revendications 3, **caractérisée en ce que** ledit anticorps est capable d'inhiber l'activité promotrice de métastase de ladite protéine CD151 au sein des cellules tumorales.

3. Utilisation selon l'une quelconque des revendications précédentes, **caractérisée en ce que** lesdits cancers consistent en les cancers du colon, du poumon, de la prostate ou du pancréas.

4. Composition **caractérisée en ce qu'**elle comprend à titre de principe actif au moins un anticorps monoclonal capable de se lier spécifiquement à la protéine CD151, sécrété par l'hybridome déposé à l'ATCC sous la référence CRL-2696, ou l'un de ses fragments fonctionnels pour son utilisation dans le traitement des tumeurs primaires.

5. Composition selon l'une quelconque des revendications 6 ou 7, **caractérisée en ce qu'**elle comprend au moins un véhicule pharmaceutiquement acceptable.

6. Composition selon l'une quelconque des revendications 6 à 8, **caractérisée en ce qu'**elle comprend, en outre, comme produit de combinaison pour une utilisation simultanée, séparée ou étalée dans le temps, au moins un agent cytotoxique/cytostatique et/ou une toxine cellulaire et/ou un radioélément.

7. Composition selon l'une quelconque des revendications 6 à 9, **caractérisée en ce qu'**elle comprend, en outre, au moins un deuxième anticorps anti-tumoral.

8. Utilisation d'une composition selon l'une quelconque des revendications 6 à 10 pour la préparation d'un médicament destiné au traitement précoce du cancer.

9. Utilisation selon la revendication 11, **caractérisée en ce que** ledit cancer est sélectionné parmi le cancer du colon, du poumon, de la prostate, et du pancréas.

## Claims

1. Use of a monoclonal antibody capable of binding specifically to the CD151 protein, secreted by the hybridoma deposited at the ATCC under reference CRL-2696, or a functional fragment thereof, in the preparation of a medicament intended for treating primary tumours for the early treatment of cancer.

2. Use according to claim 1, **characterised in that** said antibody is capable of inhibiting the metastasis-promoting activity of said CD151 protein within tumour cells.

3. Use according to either one of the preceding claims, **characterised in that** said cancers consist of colon, lung, prostate or pancreatic cancers.

4. Composition, **characterised in that** it comprises, as active ingredient, at least one monoclonal antibody capable of binding specifically to the CD151 protein, secreted by the hybridoma deposited at the ATCC under reference CRL-2696, or a functional fragment thereof, for use thereof in the treatment of primary tumours.

5. Composition according to claim 4, **characterised in that** it comprises at least one pharmaceutically acceptable carrier.

6. Composition according to either one of claims 4 and 5, **characterised in that** it additionally comprises, as a combination product for simultaneous, separate or time-staggered use, at least one cytotoxic/cytostatic agent and/or a cell toxin and/or a radioelement.

7. Composition according to any one of claims 4 to 6, **characterised in that** it additionally comprises at least one second anti-tumour antibody.

8. Use of a composition according to any one of claims 4 to 7 in the preparation of a medicament intended for the early treatment of cancer.

9. Use according to claim 8, **characterised in that** said cancer is selected from colon, lung, prostate and pancreatic cancers.

## Patentansprüche

1. Verwendung eines monoklonalen Antikörpers, der spezifisch an das CD151-Protein binden kann und der durch das bei der ATCC unter der Bezugsnummer CRL-2696 hinterlegte Hybridom sezerniert wird, oder eines seiner funktionellen Fragmente zur Herstellung eines Arzneimittels für die Behandlung primärer Tumoren zur frühzeitigen Behandlung von Krebs.

2. Verwendung nach Anspruch 1, **dadurch gekennzeichnet, dass** der Antikörper die Metastasenfördernde Aktivität des CD151-Proteins im Inneren von Tumorzellen hemmen kann.

3. Verwendung nach einem der vorstehenden Ansprüche, **dadurch gekennzeichnet, dass** die Krebserkrankungen aus Kolon-, Lungen-, Prostata- oder Pankreaskrebs bestehen.

4. Zusammensetzung, **dadurch gekennzeichnet, dass** sie als Wirkstoff mindestens einen monoklonalen Antikörper, der spezifisch an das CD151-Protein binden kann und der durch das bei der ATCC unter der Bezugsnummer CRL-2696 hinterlegte Hybridom sezerniert wird, oder eines seiner funktionellen Fragmente enthält, für die Verwendung zur Behandlung primärer Tumoren.

5. Zusammensetzung nach Anspruch 4, **dadurch gekennzeichnet, dass** sie mindestens ein pharmazeutisch annehmbares Vehikel umfasst.

6. Zusammensetzung nach einem der Ansprüche 4 oder 5, **dadurch gekennzeichnet, dass** sie außerdem als Kombinationsprodukt für eine gleichzeitige, getrennte oder zeitlich versetzte Verwendung mindestens ein zytotoxisches/zytostatisches Mittel und/oder ein Zelltoxin und/oder eine radioaktive Substanz umfasst.

7. Zusammensetzung nach einem der Ansprüche 4 bis 6, **dadurch gekennzeichnet, dass** sie außerdem mindestens einen zweiten Anti-Tumor-Antikörper umfasst.

8. Verwendung einer Zusammensetzung nach einem der Ansprüche 4 bis 7 zur Herstellung eines Arzneimittels für die frühzeitige Behandlung von Krebs.

9. Verwendung nach Anspruch 8, **dadurch gekennzeichnet, dass** der Krebs aus Kolon-, Lungen-, Prostata- oder Pankreaskrebs ausgewählt wird.
